(19) Europäisches Patentamt
European Patent Office
Office européen des brevets

(11) Veröffentlichungsnummer: **0 013 873**
**B1**

(12) # EUROPÄISCHE PATENTSCHRIFT

(45) Veröffentlichungstag der Patentschrift: **20.01.82**

(21) Anmeldenummer: **80100006.8**

(22) Anmeldetag: **02.01.80**

(51) Int. Cl.³: **C 07 D 249/08**, C 07 D 231/12, C 07 D 233/56, C 07 D 233/68, C 07 D 403/12, A 01 N 43/48, A 01 N 43/50, A 01 N 43/64

(54) **N-Azolylessigsäureanilide, Verfahren zu ihrer Herstellung und ihre Anwendung als Herbizide.**

(30) Priorität: **17.01.79 DE 2901593**

(43) Veröffentlichungstag der Anmeldung: **06.08.80 Patentblatt 80/16**

(45) Bekanntmachung des Hinweises auf die Patenterteilung: **20.01.82 Patentblatt 82/3**

(84) Benannte Vertragsstaaten: **AT BE CH DE FR GB IT LU NL SE**

(56) Entgegenhaltungen:
**EP-A-0 000 539**
**DE-A-2 643 477**
**DE-A-2 702 102**
**DE-A-2 804 299**

(73) Patentinhaber: **BASF Aktiengesellschaft, Carl-Bosch-Strasse 38, D-6700 Ludwigshafen (DE)**

(72) Erfinder: **Eicken, Karl, Dr., Waldstrasse 63, D-6706 Wachenheim (DE)**
Erfinder: **Rohr, Wolfgang, Dr., In der Dreispitz 13, D-6706 Wachenheim (DE)**
Erfinder: **Wuerzer, Bruno, Dr., Wilhelm-Busch-Strasse 55, D-6703 Limburgerhof (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG.

# N-Azolylessigsäureanilide, Verfahren zu ihrer Herstellung und ihre Anwendung als Herbizide

Die Erfindung betrifft neue N-Azolylessigsäureanilide, Verfahren zu ihrer Herstellung sowie ihre Verwendung als Herbizide.

Es ist bereits bekannt, N-Azolyl-methyl-chloressigsäureanilide als Herbizide zu verwenden (DE-OS 2648008).

Es wurde gefunden, das N-Azolylessigsäureanilide der Formel I

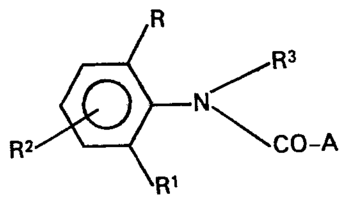

(I)

in welcher
R Wasserstoff oder $C_1$–$C_4$-Alkyl,
$R^1$ Wasserstoff, $C_1$–$C_4$-Alkyl oder Halogen,
$R^2$ Wasserstoff oder Methyl,
$R^3$ A bedeutet, und
A einen N-Azolylmethyl-Rest bedeutet, der im heterocyclischen Ring durch ein bis drei Reste aus der Gruppe Methyl, Methoxy oder Chlor substituiert sein kann,
herbizide Wirkungen haben und regulierend in das Wachstum von Pflanzen eingreifen.

Dabei vertragen Kulturpflanzen die Behandlung mit diesen Verbindungen in hohem Masse, während unerwünschte Pflanzen bekämpft werden.

Es wurde weiterhin gefunden, dass man die N-Azolylessigsäureanilide der Formel I erhält, wenn man ein 2-Halogenacetanilid der Formel II

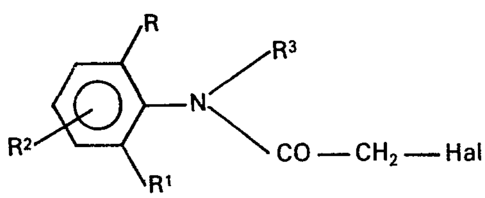

(II)

in welcher R, $R^1$, $R^2$ und $R^3$ die oben angegebene Bedeutung haben und Hal Halogen, insbesondere Chlor oder Brom bedeutet, mit einem Azol der Formel II

M–B (III)

in welcher M Wasserstoff oder ein Alkalimetall und B einen gegebenenfalls durch ein bis drei Reste aus der Gruppe Methyl, Methoxy oder Chlor substituierten Azol-Rest bedeutet, gegebenenfalls in Gegenwart eines Halogenwasserstoff bindenden Mittels und gegebenenfalls eines gegenüber den Reaktionspartnern inerten Lösungsmittels umsetzt (Verfahren A).

Es wurde weiter gefunden, dass man die oben beschriebene Umsetzung für den Fall, dass M Wasserstoff bedeutet, vorteilhaft in einem Zweiphasensystem durchführt, wobei die eine Phase aus wässrigem Alkali und die andere Phase aus einem mit der wässrigen Phase nicht mischbaren unter den Reaktionsbedingungen inerten Lösungsmittel besteht, und die Umsetzung in Gegenwart eines Phasentransferkatalysators durchführt (Verfahren B).

Das Verfahren A wird bevorzugt.

Unter Alkyl bei den Resten R, $R^1$ und $R^2$ sind je nach Zahl der genannten Kohlenstoffatome folgende Gruppen zu verstehen: Methyl, Äthyl, n-Propyl, iso-Propyl, n-, iso-, sec.- oder tert.-Butyl.

Unter Halogen sind Chlor, Brom oder Jod, insbesondere Chlor, zu verstehen. Unter dem N-Azolylmethyl-Rest A sind folgende über eine Methylgruppe am Ring-Stickstoffatom gebundenen Azole wie Pyrazol, Imidazol, 1,2,4-Triazol oder 1,2,3-Triazol sowie deren am Ringkohlenstoffatom durch ein bis drei Reste aus der Gruppe Methyl, Methoxy oder Chlor substituierten Vertreter zu verstehen, z.B. 4-Methylpyrazol, 4-Methoxypyrazol, 4-Chlorpyrazol, 3,5-Dimethylpyrazol, 3(5)-Methylpyrazol, 3(5)-Methyl-1,2,4-triazol, 3,5-Dimethyl-1,2,4-triazol, 4,5-Dimethyl-1,2,3-triazol, 2-Methylimidazol, 4,5-Dichlorimidazol, 2-Methyl-4,5-dichlorimidazol.

Die Umsetzungen (Verfahren A) können in Anwesenheit oder Abwesenheit von Lösungsmitteln durchgeführt werden, gegenüber denen die Reaktionspartner inert sind.

So eignen sich für das Verfahren A z.B. aromatische Kohlenwasserstoffe wie Toluol, Xylol; halogenierte Kohlenwasserstoffe wie Chlorbenzol, Dichlorbenzol, Tetrachlorkohlenstoff; Äther wie Tetrahydrofuran, Dioxan; Nitrile wie Acetonitril; N,N-Dialkylamide wie Dimethylformamid oder Sulfone wie Dimethylsulfoxid und auch Wasser sowie Gemische dieser Lösungsmittel, wenn M Wasserstoff bedeutet. Dabei kann der freiwerdende Halogenwasserstoff durch säurebindende Mittel beispielsweise tertiäre Amine, z.B. Triäthylamin, Pyridin und Pyridinbasen oder auch durch Einsatz von mindestens der doppelten molaren Menge an Azol H–B gebunden werden. Setzt man die Alkalisalze der Azole als Reaktionspartner ein, so ist die Verwendung von aprotisch polaren Lösungsmitteln wie N,N-Dialkylamide z.B. Dimethylformamid, wie Nitrile z.B. Acetonitril, oder wie Sulfone z.B. Dimethylsulfoxid vorteilhaft. Die Reaktionstemperaturen liegen zwischen 0 und 200 °C, vorzugsweise 25 und 150 °C. Bezogen auf eingesetztes 2-Halogenacetanilid werden mindestens molare Mengen Azol und säurebindendes Mittel bzw. mindestens molare Mengen Alkalisalz des Azols verwendet.

Nach dem Verfahren B erfolgt die Herstellung der N-Azolylessigsäureanilide der Formel I in einem Zweiphasensystem, bei dem eine Phase wässriges Alkali und die andere Phase ein mit Wasser nicht mischbares unter den Reaktionsbedingungen inertes Lösungsmittel ist, z.B. ein aromatischer Kohlenwasserstoff oder ein halogenierter Kohlenwasserstoff. Dabei wird dem Zweiphasensystem ein Phasentransferkatalysator in Mengen von 0,5 bis 30 Molprozent, bezogen auf eingesetztes 2-Halogenacetanilid der Formel II, zugesetzt. Als Phasentransferkatalysatoren kommen «Onium»-Verbindungen, z.B. quaternäre Ammonium- oder Phosphoniumverbindungen oder auch makrocyclische Polyäther in Frage. Die Reaktionstemperaturen liegen bei 0 bis 150 °C, vorzugsweise 25 bis 100 °C. Bezogen auf eingesetztes 2-Halogenacetanilid der Formel II werden mindestens molare Mengen Azol und Alkali verwendet.

Zur Isolierung der Verbindungen der Formel I wird, gegebenenfalls nach Abfiltrieren des entstandenen Halogenids, das Filtrat eingedampft und der Rückstand in einem organischen, mit Wasser nicht mischbaren Lösungsmittel aufgenommen. Nach dem Waschen mit Wasser wird die organische Phase getrocknet und danach im Vakuum eingeengt. Das so erhaltene Produkt der Formel I ist in vielen Fällen rein, wenn nötig, kann es durch Umkristallisieren aus einem geeigneten Lösungsmittel oder durch Chromatographie an Kieselgel weiter gereinigt werden.

Die als Ausgangsverbindungen verwendeten 2-Halogenacetanilide der Formel II sind bekannte Verbindungen (z.B.: DE-PS 1014380, DE-AS 2305495, DE-AS 2328340, DE-AS 1543751, DOS 2648008, DE-AS 2362743, DE-AS 1542702).

Die folgenden Beispiele erläutern die Herstellung der neuen N-Azolylessigsäureanilide, ohne die erfindungsgemässen Verfahren einzuschränken. In den Beispielen verhalten sich Gewichtsteile zu Volumenteilen wie Kilogramm zu Liter.

Beispiel 1: (Verfahren B)

27,8 Gewichtsteile 2-Chlor-2',6'-dimethyl-N-(pyrazol-1-yl-methyl)-acetanilid gelöst in 50 Volumenteilen Toluol sowie 81 Gewichtsteile Pyrazol, 4,0 Gewichtsteile Natriumhydroxid und 2,0 Gewichtsteile Benzyltriäthylammoniumchlorid gelöst in 20 Volumenteilen Wasser werden 8 Stunden bei 80 °C intensiv vermischt. Die organische Phase wird danach abgetrennt, mit 60 Volumenteilen Methylenchlorid versetzt, dreimal mit je 100 Volumenteilen Wasser gewaschen und getrocknet über Natriumsulfat. Nach dem Verdampfen der Lösungsmittel im Vakuum und Umkristallisieren des Rückstands aus 40 Volumenteilen Toluol isoliert man 25,0 Gewichtsteile N-(Pyrazol-1-yl-methyl)-pyrazol-1-yl-essigsäure-2',6'-dimethylanilid vom Fp. 133 °C.

$C_{17}H_{19}N_5O$ (M 309)
ber.: C 66,0, H 6,1, N 22,6%;
gef.: C 66,1, H 5,8, N 22,4%.

Beispiel 2: (Verfahren A)

13,8 Gewichtsteile 2-Chlor-2',6'-dimethyl-N-(pyrazol-1-yl-methyl)-acetanilid, 17,0 Gewichtsteile Imidazol, 15 Volumenteile Dioxan, 50 Volumenteile Wasser werden 20 Stunden unter Rückfluss erhitzt und dann im Vakuum eingeengt. Der Rückstand wird dreimal mit je 80 Volumenteilen Essigester extrahiert. Nach dem Waschen der vereinigten organischen Phasen mit 100 Volumenteilen Wasser wird die Lösung über eine Säule mit Kieselgel filtriert. Aus dem Eluat isoliert man nach dem Verdampfen des Lösungsmittels 7,5 Gewichtsteile N-(Pyrazol-1-yl-methyl)-imidazol-1-yl-essigsäure-2',6'-dimethylanilid als Öl ($n_D^{25}$ = 1,5505).

$C_{17}H_{19}N_5O$ (M 309).

In entsprechender Weise (Verfahren A oder B) werden die folgenden Verbindungen hergestellt:

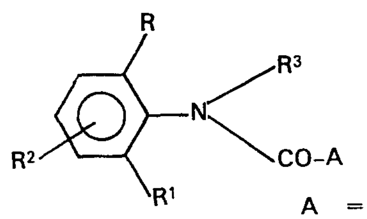

A = $-CH_2-R^4$

| Verb. Nr. | R | $R^1$ | $R^2$ | $R^3$ | $R^4$ | Fp, $n_D^{25}$, Kp |
|---|---|---|---|---|---|---|
| 1 | H | H | H | 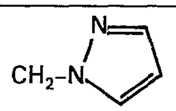 | 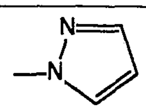 | 82 |
| 2 | H | H | H | 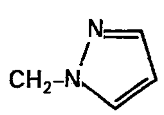 | 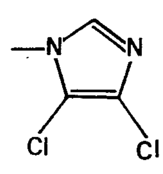 | 150 |
| 3 | H | H | H | 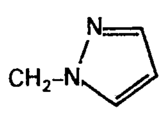 | 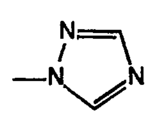 | |
| 4 | H | H | H | 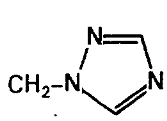 | 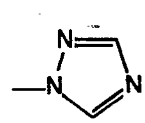 | |

| Verb. Nr. | R | $R^1$ | $R^2$ | $R^3$ | $R^4$ | Fp, $n_D^{25}$, Kp |
|---|---|---|---|---|---|---|
| 5 | H | H | H | 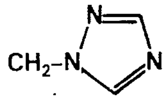 | 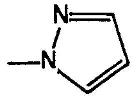 | |
| 6 | $CH_3$ | $CH_3$ | H | 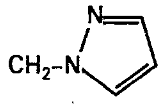 | 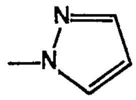 | 133 |
| 7 | $CH_3$ | $CH_3$ | H | 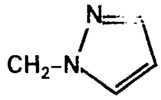 | 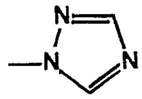 | viskos |
| 8 | $CH_3$ | $CH_3$ | H | 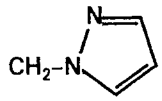 | 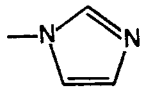 | 1,5505 |
| 9 | $CH_3$ | $CH_3$ | H | 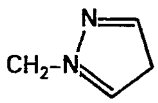 | 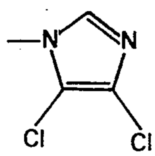 | 179 |
| 10 | $CH_3$ | $CH_3$ | 3-$CH_3$ | 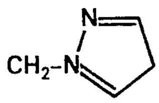 | 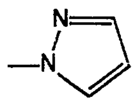 | |
| 11 | $CH_3$ | $CH_3$ | 3-$CH_3$ | 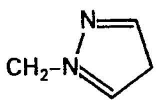 | 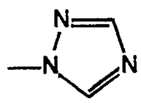 | |
| 12 | $CH_3$ | $CH_3$ | H | 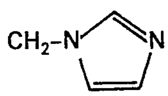 | 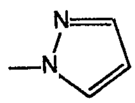 | |
| 13 | $CH_3$ | $CH_3$ | H | 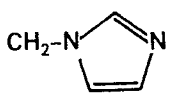 | 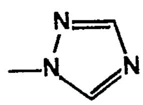 | |
| 14 | $CH_3$ | $CH_3$ | H | 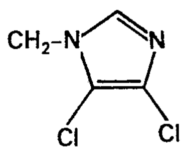 | 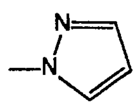 | |
| 15 | $CH_3$ | $CH_3$ | H | 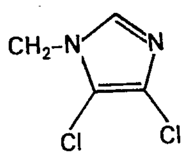 | 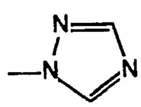 | |
| 16 | $CH_3$ | $C_2H_5$ | H | 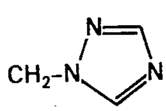 | 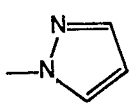 | 122 |

| Verb. Nr. | R | $R^1$ | $R^2$ | $R^3$ | $R^4$ | Fp, $n_D^{25}$, Kp |
|---|---|---|---|---|---|---|
| 17 | $CH_3$ | $C_2H_5$ | H | $CH_2$-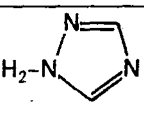 | 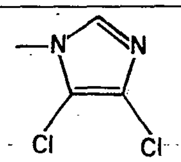 | 115 |
| 18 | $CH_3$ | $C_2H_5$ | H | $CH_2$-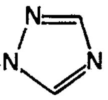 | 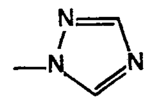 | |
| 19 | $CH_3$ | $C_2H_5$ | H | $CH_2$-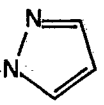 | 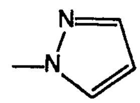 | |
| 20 | $CH_3$ | $C_2H_5$ | H | $CH_2$-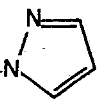 | 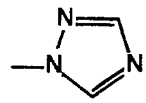 | |
| 21 | $C_2H_5$ | $C_2H_5$ | H | $CH_2$-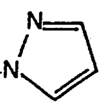 | 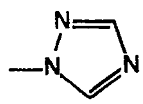 | |

Die neuen Wirkstoffe werden beispielsweise in Form von direkt versprühbaren Lösungen, Pulvern, Suspensionen, auch hochprozentigen wässrigen, öligen oder sonstigen Suspensionen oder Dispersionen, Emulsionen, Öldispersionen, Pasten, Stäubemitteln, Streumitteln, Granulaten durch Versprühen, Vernebeln, Verstäuben, Verstreuen oder Giessen angewendet. Die Anwendungsformen richten sich ganz nach den Verwendungszwecken; sie sollten in jedem Fall möglichst die feinste Verteilung der erfindungsgemässen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Diselöl, ferner Kohlenteeröle, sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate zum Beispiel Methanol, Äthanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser in Betracht.

Wässrige Anwendungsformen können aus Emulsionskonzentraten, Pasten oder benetzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünung mit Wasser geeignet sind.

Als oberflächenaktive Stoffe kommen in Betracht: Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylen-octylphenoläther, äthoxyliertes Isooctylphenol-, Octylphenol-, Nonylphenol, Alkylphenolpolyglykoläther, Tributylphenylpolyglykoläther, Alkalarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löss, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nusschalenmehl, Cellulosepulver und andere feste Trägerstoffe.

Die Formulierungen enthalten zwischen 0,1 und 95 Gewichtsprozent Wirkstoff, vorzugsweise zwischen 0,5 und 90 Gewichtsprozent.

Der Einfluss der neuen Verbindungen auf das Wachstum von Pflanzen wird in folgenden Gewächshausversuchen demonstriert.

Als Kulturgefässe dienten Plastikblumentöpfe mit 300 $cm^3$ Inhalt und lehmiger Sand mit etwa 1,5% Humus als Substrat.

Die Samen der Testpflanzen entsprechend Tabelle 1 wurden nach Arten getrennt flach eingesät. Bei Cyperus esculentus nahm man vorgekeimte Knollen. Unmittelbar danach erfolgte bei Vorauflaufbehandlung das Aufbringen der Wirkstoffe auf die Erdoberfläche. Die Wirkstoffe wurden hierbei in Wasser als Verteilungsmittel suspendiert oder emulgiert und mittels fein verteilender Düsen gespritzt. Nach dem Aufbringen der Mittel wurden die Gefässe leicht beregnet, um Keimung und Wachstum in Gang zu bringen und auch gleichzeitig die Wirkstoffe zu aktivieren. Danach deckte man die Gefässe mit durchsichtigen Plastikhauben ab, bis die Pflanzen angewachsen waren. Diese Abdeckung bewirkte ein gleichmässiges Keimen der Testpflanzen, sofern dies nicht durch die Wirkstoffe beeinträchtigt wurde, und verhinderte das Verdampfen leicht flüchtiger Substanzen.

Zum Zwecke der Nachauflaufbehandlung zog man die Pflanzen je nach Wuchsform in den Versuchsgefässen erst bis zu einer Höhe von 3 bis 10 cm an und behandelte sie danach. Eine Abdekkung unterblieb. Die Aufstellung der Versuche erfolgte im Gewächshaus, wobei für wärmeliebende Arten wärmere Bereiche (25 bis 40 °C) und für solche gemässigter Klimate 15 bis 30 °C bevorzugt wurden. Die Versuchsperiode erstreckte sich über 4 Wochen. Während dieser Zeit wurden die Pflanzen gepflegt und ihre Reaktion auf die einzelnen Behandlungen wurde ausgewertet. Die folgenden Tabellen enthalten die Prüfsubstanzen, die jeweiligen Dosierungen in kg/ha Aktivsubstanz und die Testpflanzenarten. Bewertet wird nach einer Skala von 0 bis 100. Dabei bedeutet 0 keine Schädigung oder normaler Ablauf und 100 kein Aufgang der Pflanzen bzw. völlige Zerstörung zumindest der oberirdischen Sprossteile.

Die beigefügten Tabellen zeigen die Resultate. Die erfindungsgemässen Verbindungen wurden hier bei Vor- und Nachauflaufbehandlung geprüft. Gerade die Nachauflaufanwendung lieferte neben der Wirkung auf andere unerwünschte Pflanzen einen beachtlichen Bekämpfungserfolg gegen Cyperus esculentus, ein landwirtschaftlich enorm wichtiges Unkraut, ohne dass bei den verwendeten Aufwandmengen bedeutsame landwirtschaftliche Kulturpflanzen Schaden erlitten.

Neben der Oberflächenspritzung (surface application) können die Wirkstoffe auch in den Boden eingearbeitet werden. Dabei kann es sich bei Kulturpflanzenbeständen um eine Einarbeitung vor der Saat, nach der Saat oder zwischen den bereits vorhandenen Nutzpflanzen handeln.

Sind gegenüber den Wirkstoffen empfindliche Kulturpflanzen vorhanden, so können auch Ausbringungstechniken angewandt werden, bei welchen die Mittel mit Hilfe der Spritzgeräte so gespritzt werden, dass die Blätter empfindlicher Kulturpflanzen nach Möglichkeit nicht getroffen werden, während sie auf die darunter liegende Bodenfläche oder dort wachsenden unerwünschten Pflanzen gelangen (post-directed, lay-by).

Weiter können die neuen Wirkstoffe auf Flächen angewendet werden, bei denen Graswuchs lediglich im Wachstum gebremst werden soll, ohne die Pflanzen abzutöten.

In Anbetracht der Vielseitigkeit der Applikationsmethoden können die erfindungsgemässen Mittel oder diese enthaltende Mischungen ausser bei den in den Tabellen aufgeführten Nutzpflanzen noch in einer weiteren grossen Zahl von Kulturpflanzen zur Beseitigung oder Unterdrückung unerwünschten Pflanzenwuchses eingesetzt werden. Die Aufwandmengen können dabei von 0,1 bis 15 kg/ha und mehr je nach dem Bekämpfungsziel schwanken.

Im einzelnen seien folgende Nutzpflanzen genannt:

| Botanischer Name | Deutscher Name | Englischer Name |
|---|---|---|
| Allium cepa | Küchenzwiebel | onions |
| Ananas comosus | Ananas | pineapple |
| Arachis hypogaea | Erdnuss | peanuts (groundnuts) |
| Asparagus officinalis | Spargel | asparagus |
| Avena sativa | Hafer | oats |
| Beta vulgaris spp. altissima | Zuckerrübe | sugarbeets |
| Beta vulgaris spp. rapa | Futterrübe | fodder beets |
| Beta vulgaris spp. esculenta | Rote Rübe | table beets, red beets |
| Brassica napus var. napus | Raps | rape seed |
| Brassica napus var. napobrassica | Kohlrübe | |
| Brassica napus var. rapa | Weisse Rübe | turnips |
| Brassica rapa var. silvestris | Rübsen | |
| Camellia sinensis | Teestrauch | tea plants |
| Carthamus tinctorius | Saflor - Färberdistel | safflower |

| Botanischer Name | Deutscher Name | Englischer Name |
|---|---|---|
| Carva illinoinensis | Pekannussbaum | pecan trees |
| Citrus limon | Zitrone | lemon |
| Citrus maxima | Pampelmuse | grapefruits |
| Citrus reticulata | Mandarine | |
| Citrus sinensis | Apfelsine, Orange | orange trees |
| Coffea arabica (Coffea canephora, Coffea liberica) | Kaffee | coffee plants |
| Cucumis melo | Melone | melons |
| Cucumis sativus | Gurke | cucumber |
| Cynodon dactylon | Bermudagras | Bermudagrass in turfs and lawns |
| Daucus carota | Möhre | carrots |
| Elaeis guineensis | Ölpalme | oil palms |
| Fragaria vesca | Erdbeere | strawberries |
| Glycine max | Sojabohne | soybeans |
| Gossypium hirsutum (Gossypium arboreum, Gossypium herbaceum, Gossypium vitifolium) | Baumwolle | cotton |
| Helianthus annuus | Sonnenblume | sunflowers |
| Helianthus tuberosus | Topinambur | |
| Hevea brasiliensis | Parakautschukbaum | rubber plants |
| Hordeum vulgare | Gerste | barley |
| Humulus lupulus | Hopfen | hop |
| Ipomoea batatas | Süsskartoffeln | sweet potato |
| Juglans regia | Walnussbaum | walnut trees |
| Lactuca sativa | Kopfsalat | lettuce |
| Lens culinaris | Linse | lentils |
| Linum usitatissimum | Faserlein | flax |
| Lycopersicon lycopersicum | Tomate | tomato |
| Malus spp. | Apfel | apple trees |
| Manihot esculenta | Maniok | cassava |
| Medicago sativa | Luzerne | alfalfa (lucerne) |
| Mentha piperita | Pfefferminze | peppermint |
| Musa spp. | Obst- und Mehlbanane | banana plants |
| Nicotiana tabacum (N. rustica) | Tabak | tabacco |
| Olea europaea | Ölbaum | olive trees |
| Oryza sativa | Reis | rice |
| Panicum miliaceum | Rispenhirse | |
| Phaseolus lunatus | Mondbohne | limabeans |
| Phaseolus mungo | Urdbohne | mungbeans |
| Phaseolus vulgaris | Buschbohnen | snapbeans, green beans, dry beans |
| Pennisetum glaucum | Perl- oder Rohrkolbenhirse | |
| Petroselinum crispum spp. tuberosum | Wurzelpetersilie | parsley |
| Picea abies | Rotfichte | Norway spruce |
| Abies alba | Weisstanne | fire |
| Pinus spp. | Kiefer | pine trees |
| Pisum sativum | Gartenerbse | English peas |
| Prunus avium | Süsskirsche | cherry trees |
| Prunus domestica | Pflaume | plum trees |
| Prunus dulcis | Mandelbaum | almond trees |
| Prunus persica | Pfirsich | peach trees |
| Pyrus communis | Birne | pear trees |
| Ribes sylvestre | Rote Johannisbeere | red currants |
| Ribes uva-crispa | Stachelbeere | |
| Ricinus communis | Rizinus | |
| Saccharum officinarum | Zuckerrohr | sugar cane |
| Secale cereale | Roggen | rye |
| Sesamum indicum | Sesam | Sesame |
| Solanum tuberosum | Kartoffel | Irish potatoes |
| Sorghum bicolor (s. vulgare) | Mohrenhirse | sorghum |
| Sorghum dochna | Zuckerhirse | |
| Spinacia oleracea | Spinat | spinach |

| Botanischer Name | Deutscher Name | Englischer Name |
|---|---|---|
| Theobroma cacao | Kakaobaum | cacao plants |
| Trifolium pratense | Rotklee | red clover |
| Triticum aestivum | Weizen | wheat |
| Vaccinium corymbosum | Kulturheidelbeere | blueberry |
| Vaccinium vitis-idaea | Preisselbeere | cranberry |
| Vicia faba | Pferdebohnen | tick beans |
| Vigna sinensis (V. unguiculata) | Kuhbohne | cow peas |
| Vitis vinifera | Weinrebe | grapes |
| Zea mays | Mais | Indian corn, sweet corn, maize |

Die neuen Verbindungen können auch als wachstumsregulierende Mittel für Pflanzen, beispielsweise zur Reduzierung des Längenwachstums, verwendet werden. Die Aufwandmengen betragen hierbei beispielsweise 0,01 bis 10 kg/ha.

Die neuen erfindungsgemässen Acetanilide können unter sich gemischt, in Kombination mit bekannten Halogenacetaniliden und in Mischung mit zahlreichen Vertretern anderer herbizider oder wachstumsregulierender Wirkstoffgruppen gemeinsam ausgebracht werden. Solche Kombinationen dienen zur Verbreiterung des Wirkungsspektrums und erzielen zuweilen synergistische Effekte.

Tabelle 1 - Liste der Pflanzennamen

| Botanische Bezeichnung | Abkürzung in Tabellen | Deutscher Name | Englische Bezeichnung |
|---|---|---|---|
| Alopecurus myosuroides | - | Ackerfuchsschwanz | slender foxtail |
| Amaranthus retroflexus | - | zurückgekrümmter Fuchsschwanz | redroot pigweed |
| Beta vulgaris | Beta vulg. | Zuckerrübe | sugar beet |
| Brassica napus | - | Raps | rape seed |
| Bromus tectorum | Bromus tect. | Dach-Trespe | downy broome |
| Cyperus esculentus | Cyper. escul. | Erdmandel | yellow nutsedge |
| Echinochloa crus galli | Echin. c.g. | Hühnerhirse | barnyard grass |
| Gossypium hirsutum | Gossyp. hirs. | Baumwolle | cotton |
| Sorghum halepense | Sorg. halep. | Sudangras | Johnsongrass |

Tabelle 2 - Selektive Bekämpfung unerwünschter Gräser in Zuckerrüben bei Vorauflaufanwendung im Gewächshaus

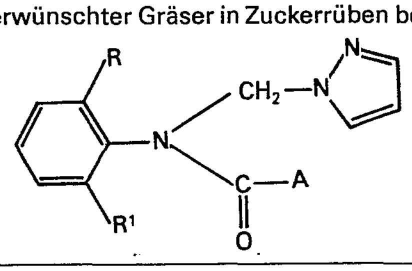

| Wirkstoff Nr. | Substituenten A | R | $R^1$ | kg/ha a.S. | Testpflanzen und % Schädigung: Beta vulgaris | Alopecurus myosuroides | Bromus tectorum | Echinochloa crus galli | Amaranthus retroflexus |
|---|---|---|---|---|---|---|---|---|---|
| 7 | $-CH_2$-1,2,4-triazolyl-1 | $CH_3$ | $CH_3$ | 1,0 | 0 | 90 | 90 | 99 | 95 |
| 9 | $-CH_2$-4,5-dichlor-imidazolyl-1 | $CH_3$ | $CH_3$ | 2,0 | 0 | - | — | 98 | 85 |
| 6 | $-CH_2$-pyrazolyl-1 | $CH_3$ | $CH_3$ | 2,0 | 10 | - | 100 | 83 | 97 |
| bekannt (DE-OS 26 48 008) | $CH_2Cl$ | $CH_3$ | $CH_3$ | 1,0 | 85 | 100 | 100 | 100 | 100 |

0 = keine Schädigung
100 = Pflanzen nicht aufgelaufen bzw. abgestorben

Tabelle 3 – Unterdrückung von Cyperus und unerwünschten Gräsern bei Nachauflaufanwendung im Gewächshaus

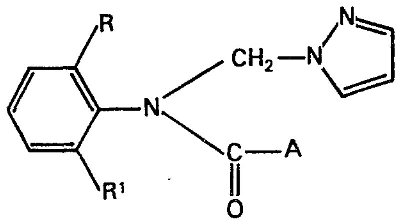

| Wirkstoff Nr. | Substituenten A | R | $R^1$ | kg/ha a.S. | Testpflanzen und % Schädigung Beta vulg. | Brassica napus | Gossyp. hirs. | Cyper. escul. | Bromus tect. | Echin. c.g. | Sorgh. halep.* |
|---|---|---|---|---|---|---|---|---|---|---|---|
| 7 | $-CH_2-$ -1,2,4- -triazolyl-1 | $CH_3$ | $CH_3$ | 1,0 | 0 | 0 | 0 | 85 | 70 | 75 | 95 |
| | | | | 2,0 | 0 | 0 | 0 | 85 | 80 | 95 | 95 |
| bekannt (DE-OS 26 48 008) | $CH_2Cl$ | $CH_3$ | $CH_3$ | 1,0 | 35 | 20 | 18 | 75 | 80 | 92 | 89 |

* aus Samen; 0 = keine Schädigung; 100 = Pflanzen nicht aufgelaufen bzw. abgestorben.

Tabelle 4 – Herbizide Wirkung bei Vorauflaufbehandlung im Gewächshaus

| Wirkstoff Nr. | kg/ha | Testpflanzen und Schädigung % Echinochloa crus galli |
|---|---|---|
| 16 | 3,0 | 100 |

Beispiel I

Man vermischt 90 Gewichtsteile der Verbindung 1 mit 10 Gewichtsteilen N-Methyl-α-pyrrolidon und erhält eine Lösung, die zur Anwendung in Form kleinster Tropfen geeignet ist.

Beispiel II

20 Gewichtsteile der Verbindung 2 werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-monoäthanolamid, 5 Gewichtsteilen Calciumsalz der Dodecylbenzolsulfonsäure und 5 Gewichtsteile des Anlagerungsproduktes von 40 Mol Äthylenoxid an 1 Mol Rizinusöl besteht. Durch Ausgiessen und feines Verteilen der Lösung in 100 000 Gewichtsteilen Wasser erhält man eine wässrige Dispersion, die 0,02 Gewichtsprozent des Wirkstoffs enthält.

Beispiel III

20 Gewichtsteile der Verbindung 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Rizinusöl besteht. Durch Eingiessen und feines Verteilen der Lösung in 100 000 Gewichtsteilen Wasser erhält man eine wässrige Dispersion, die 0,02 Gewichtsprozent des Wirkstoffs enthält.

Beispiel IV

20 Gewichtsteile der Verbindung 1 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Äthylenoxid an 1 Mol Rizinusöl besteht. Durch Eingiessen und feines Verteilen der Lösung in 100 000 Gewichtsteilen Wasser erhält man eine wässrige Dispersion, die 0,02 Gewichtsprozent des Wirkstoffs enthält.

Beispiel V

20 Gewichtsteile des Wirkstoffs 2 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

Beispiel VI

3 Gewichtsteile der Verbindung 3 werden 97 Gewichtsteilen feinteiligem Kaolin innig vermischt. Man erhält auf diese Weise ein Stäubemittel, das 3 Gewichtsprozent des Wirkstoffs enthält.

Beispiel VII

30 Gewichtsteile der Verbindung 4 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufarbeitung des Wirkstoffs mit guter Haftfähigkeit.

Beispiel VIII

40 Gewichtsteile des Wirkstoffs 1 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wässrige Dispersion. Durch Verdünnen mit 100000 Gewichtsteilen Wasser erhält man eine wässrige Dispersion, die 0,04 Gewichtsprozent Wirkstoff enthält.

Beispiel IX

20 Teile des Wirkstoffs 2 werden mit 2 Teilen Calciumsalz der Dodecylbenzolsulfonsäure, 8 Teilen Fettalkohol-polyglykoläther, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

**Patentansprüche** für die Vertragsstaaten: BE, CH, DE, FR, GB, IT, LU, NL, SE

1. N-Azolylessigsäureanilid der Formel I

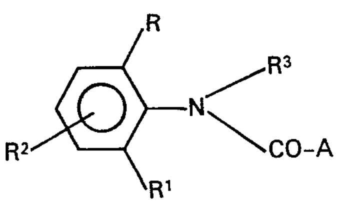

(I)

in welcher

R Wasserstoff oder $C_1$–$C_4$-Alkyl,

$R^1$ Wasserstoff, $C_1$–$C_4$-Alkyl oder Halogen,

$R^2$ Wasserstoff oder Methyl,

$R^3$ A bedeutet, und

A einen N-Azolylmethyl-Rest bedeutet, der im heterocyclischen Ring durch ein bis drei Reste aus der Gruppe Methyl, Methoxy oder Chlor substituiert sein kann.

2. Herbizid, enthaltend ein N-Azolylessigsäureanilid gemäss Anspruch 1 als Wirkstoffkomponente.

3. Herbizid, enthaltend einen festen oder flüssigen Trägerstoff und ein N-Azolylessigsäureanilid gemäss Anspruch 1.

4. Verfahren zur Herstellung eines Herbizids, dadurch gekennzeichnet, dass man einen festen oder flüssigen Trägerstoff vermischt mit einem N-Azolylessigsäureanlid gemäss Anspruch 1.

5. Verfahren zur Bekämpfung von unerwünschtem Pflanzenwuchs, dadurch gekennzeichnet, dass man den Boden oder die Pflanzen behandelt mit einem N-Azolylessigsäureanilid gemäss Anspruch 1.

6. Verfahren zur Herstellung eines N-Azolylessigsäureanilids gemäss Anspruch 1, dadurch gekennzeichnet, dass man ein 2-Halogenacetanilid der Formel II

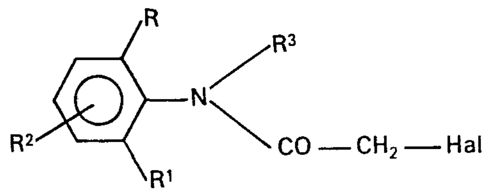

(II)

in welcher R, $R^1$, $R^2$ und $R^3$ die im Anspruch 1 genannte Bedeutung haben und Hal Halogen, insbesondere Chlor oder Brom bedeutet, mit einem Azol der Formel III

M–B (III)

in welcher M Wasserstoff oder ein Alkalimetall und B einen durch ein bis drei Reste aus der Gruppe Methyl, Methoxy oder Chlor substituierten Azol-Rest bedeutet, gegebenenfalls in Gegenwart eines halogenwasserstoffbindenden Mittels und gegebenenfalls eines gegenüber den Reaktionspartnern inerten Lösungsmittels umsetzt.

7. Verfahren zur Herstellung eines N-Azolylessigsäureanilids gemäss Anspruch 6, dadurch gekennzeichnet, dass man die Umsetzung in einem Zweiphasensystem durchführt, wobei die eine Phase aus wässrigem Alkali und die andere Phase aus einem mit der wässrigen Phase nicht mischbaren unter den Reaktionsbedingungen inerten Lösungsmittel besteht, und die Umsetzung in Gegenwart eines Phasentransferkatalysators durchführt.

8. N-Azolylessigsäureanilid gemäss Anspruch 1, nämlich
N-(1,2,4-Triazol-1-yl-methyl)-pyrazol-1-yl-essigsäure-2′,6′-dimethylanilid oder
N-(Pyrazol-1-yl-methyl)-pyrazol-1-yl-essigsäure-2′,6′-dimethylanilid.

9. Herbizid gemäss Anspruch 2, enthaltend ein N-Azolylessigsäureanilid gemäss Anspruch 1, nämlich
N-(1,2,4-Triazol-1-yl-methyl)-pyrazol-1-yl-essigsäure-2′,6′-dimethylanilid oder
N-(Pyrazol-1-yl-methyl)-pyrazol-1-yl-essigsäure-2′,6′-dimethylanilid als Wirkstoffkomponente.

**Patentansprüche** für den Vertragsstaat: AT

1. Herbizid, enthaltend ein N-Azolylessigsäureanilid der Formel I als Wirkstoffkomponente

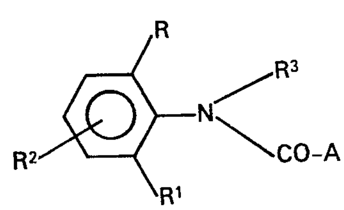

(I)

in welcher

R Wasserstoff oder $C_1$–$C_4$-Alkyl,

$R^1$ Wasserstoff, $C_1$–$C_4$-Alkyl oder Halogen,

$R^2$ Wasserstoff oder Methyl,

$R^3$ A bedeutet, und

A einen N-Azolylmethyl-Rest bedeutet, der im heterocyclischen Ring durch ein bis drei Reste aus der Gruppe Methyl, Methoxy oder Chlor substituiert sein kann.

2. Herbizid, enthaltend einen festen oder flüssigen Trägerstoff und ein N-Azolylessigsäureanilid der Formel I wie im Anspruch 1 definiert.

3. Verfahren zur Herstellung eines Herbizids, dadurch gekennzeichnet, dass man einen festen oder flüssigen Trägerstoff vermischt mit einem N-Azolylessigsäureanilid der Formel I wie im Anspruch 1 definiert.

4. Verfahren zur Bekämpfung von unerwünschtem Pflanzenwuchs, dadurch gekennzeichnet, dass man den Boden oder die Pflanzen behandelt mit einem N-Azolylessigsäureanilid der Formel I wie im Anspruch 1 definiert.

5. Verfahren zur Herstellung eines N-Azolylessigsäureanilids der Formel I wie im Anspruch 1 definiert, dadurch gekennzeichnet, dass man ein 2-Halogenacetanilid der Formel II

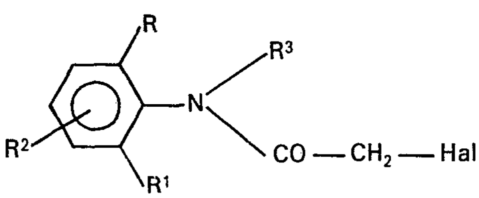

(II)

in welcher R, $R^1$, $R^2$ und $R^3$ die im Anspruch 1 genannte Bedeutung haben und Hal Halogen, insbesondere Chlor oder Brom bedeutet, mit einem Azol der Formel III

M–B (III)

in welcher M Wasserstoff oder ein Alkalimetall und B einen durch ein bis drei Reste aus der Gruppe Methyl, Methoxy oder Chlor substituierten Azol-Rest bedeutet, gegebenenfalls in Gegenwart eines halogenwasserstoffbindenden Mittels und gegebenenfalls eines gegenüber den Reaktionspartnern inerten Lösungsmittels umsetzt.

6. Verfahren zur Herstellung eines N-Azolylessigsäureanilids gemäss Anspruch 5, dadurch gekennzeichnet, dass man die Umsetzung in einem Zweiphasensystem durchführt, wobei die eine Phase aus wässrigem Alkali und die andere Phase aus einem mit der wässrigen Phase nicht mischbaren unter den Reaktionsbedingungen inerten Lösungsmittel besteht, und die Umsetzung in Gegenwart eines Phasentransferkatalysators durchführt.

7. Herbizid gemäss Anspruch 1, enthaltend ein N-Azolylessigsäureanilid als Wirkstoffkomponente, nämlich

N-(1,2,4-Triazol-1-yl-methyl)-pyrazol-1-yl-essigsäure-2′,6′-dimethylanilid oder

N-(Pyrazol-1-methyl)-pyrazol-1-yl-essigsäure-2′,6′-dimethylanilid.

**Claims** for the Contracting states: BE, CH, DE, FR, GB, IT, LU, NL, SE

1. An N-azolylacetanilide of the formula I

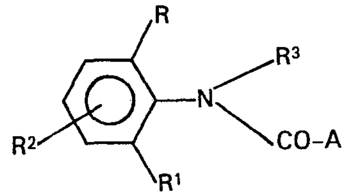

(I)

where R denotes hydrogen or $C_1$–$C_4$-alkyl, $R^1$ denotes hydrogen, $C_1$–$C_4$-alkyl or halogen, $R^2$ denotes hydrogen or methyl, $R^3$ denotes A, and A denotes N-azolylmethyl which is unsubstituted or substituted in the heterocyclic ring by from one to three radicals selected from the group consisting of methyl, methoxy and chlorine.

2. A herbicide containing an N-azolylacetanilide as claimed in claim 1 as the active ingredient.

3. A herbicide containing a solid or liquid carrier and an N-azolylacetanilide as claimed in claim 1.

4. A process for the manufacture of a herbicide, characterized in that a solid or liquid carrier is mixed with an N-azolylacetanilide as claimed in claim 1.

5. A process for controlling unwanted plant growth, characterized in that the plants or the soil are treated with an N-azolylacetanilide as claimed in claim 1.

6. A process for the manufacture of an N-azolylacetanilide as claimed in claim 1, characterized in that a 2-haloacetanilide of the formula II

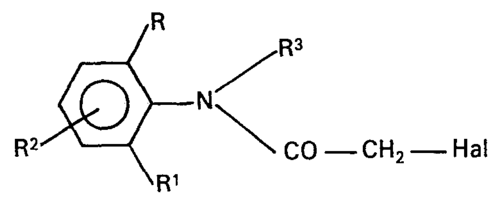

(II)

where R, $R^1$, $R^2$ and $R^3$ have the meanings given in claim 1 and Hal denotes halogen, especially chlorine or bromine, is reacted with an azole of the formula III

M–B (III),

where M denotes hydrogen or an alkali metal and B denotes azole which is substituted by from one to three radicals selected from the group consisting of methyl, methoxy and chlorine, in the presence or absence of an agent which binds hydrogen halide and in the presence or absence of a solvent inert to the reactants.

7. A process for the manufacture of an N-azolylacetanilide as claimed in claim 6, characterized in that the reaction is carried out in a 2-phase system, one phase being aqueous alkali and the other a solvent immiscible with the aqueous phase and inert under the reaction conditions, in the presence of a phase transfer catalyst.

8. An N-azolylacetanilide as claimed in claim 1, namely N-(1,2,4-triazol-1-yl-methyl)-pyrazol-1-yl-

acetic acid-2',6'-dimethylanilide or N-(pyrazol-1-yl-methyl)-pyrazol-1-yl-acetic acid-2',6'-dimethylanilide.

9. A herbicide as claimed in claim 2 containing an N-azolylacetanilide as claimed in claim 1, namely N-(1,2,4-triazol-1-yl-methyl)-pyrazol-1-yl-acetic acid-2',6'-dimethylanilide or N-(pyrazol-1-yl-methyl)-pyrazol-1-yl-acetic acid-2',6'-dimethylanilide, as active ingredient.

**Claims** for the Contracting state: AT

1. A herbicide containing an N-azolylacetanilide of the formula I as active ingredient

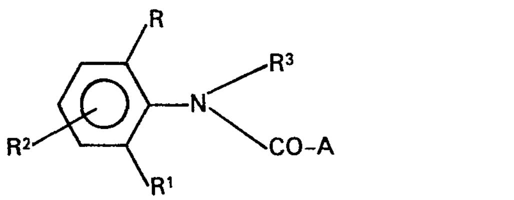 (I)

where R denotes hydrogen or $C_1$–$C_4$-alkyl, $R^1$ denotes hydrogen, $C_1$–$C_4$-alkyl or halogen, $R^2$ denotes hydrogen or methyl, $R^3$ denotes A, and A denotes N-azolylmethyl which is unsubstituted or substituted in the heterocyclic ring by from one to three radicals selected from the group consisting of methyl, methoxy and chlorine.

2. A herbicide containing a solid or liquid carrier and an N-azolylacetanilide of the formula I as defined in claim 1.

3. A process for the manufacture of a herbicide, characterized in that a solid or liquid carrier is mixed with an N-azolylacetanilide of the formula I as defined in claim 1.

4. A process for controlling unwanted plant growth, characterized in that the plants or the soil are treated with an N-azolylacetanilide of the formula I as defined in claim 1.

5. A process for the manufacture of an N-azolylacetanilide of the formula I as defined in claim 1, characterized in that a 2-haloacetanilide of the formula II

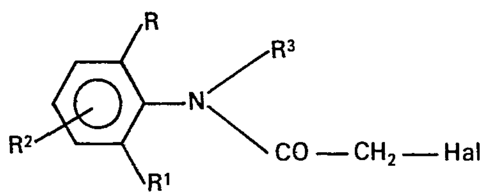 (II)

where R, $R^1$, $R^2$ and $R^3$ have the meanings given in claim 1 and Hal denotes halogen, especially chlorine or bromine, is reacted with an azole of the formula III

M–B (III),

where M denotes hydrogen or an alkali metal and B denotes azole which is substituted by from one to three radicals selected from the group consisting of methyl, methoxy and chlorine, in the presence or absence of an agent which binds hydrogen halide and in the presence or absence of a solvent inert to the reactants.

6. A process for the manufacture of an N-azolylacetanilide as claimed in claim 5, characterized in that the reaction is carried out in a 2-phase system, one phase being aqueous alkali and the other a solvent immiscible with the aqueous phase and inert under the reaction conditions, in the presence of a phase transfer catalyst.

7. A herbicide as claimed in claim 1 containing an N-azolylacetanilide as active ingredient, namely N-(1,2,4-triazol-1-yl-methyl)-pyrazol-1-yl-acetic acid-2',6'-dimethylanilide or N-(pyrazol-1-yl-methyl)-pyrazol-1-yl-acetic acid-2',6'-dimethylanilide.

**Revendications** pour les Etats contractants: BE, CH, DE, FR, GB, IT, LU, NL, SE

1. Anilide d'acide N-azolylacétique de formule I

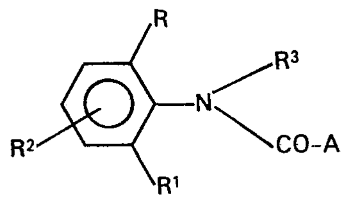 (I)

dans laquelle
R représente hydrogène ou alkyle en $C_1$ à $C_4$,
$R^1$ hydrogène, alkyle en $C_1$ à $C_4$ ou halogène,
$R^2$ hydrogène ou méthyle
$R^3$ représente A, et
A représente un reste N-azolylméthyle qui peut être substitué dans le noyau hétérocyclique par un à trois restes du groupe méthyle, méthoxy ou chlore.

2. Herbicide contenant, comme composant actif, un anilide d'acide N-azolylacétique selon la revendication 1.

3. Herbicide contenant un support liquide ou solide et un anilide d'acide N-azolylacétique selon la revendication 1.

4. Procédé de préparation d'un herbicide, caractérisé par le fait qu'on mélange un support solide ou liquide avec un anilide d'acide N-azolylacétique selon la revendication 1.

5. Procédé de lutte contre la croissance des plantes indésirables, caractérisé par le fait qu'on traite le sol ou les plantes avec un anilide d'acide N-azolylacétique selon la revendication 1.

6. Procédé de préparation d'un anilide d'acide N-azolylacétique selon la revendication 1, caractérisé par le fait qu'on fait réagir un 2-halogènacétanilide de formule II

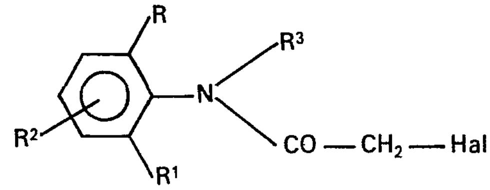 (II)

dans laquelle R, $R^1$, $R^2$ et $R^3$ ont la signification donnée à la revendication 1 et Hal représente halogène, en particulier chlore ou brome, avec un azole de formule III

M–B (III)

dans laquelle M représente hydrogène ou un métal alcalin et B un reste azole substitué par un à trois restes du groupe méthyle, méthoxy ou chlore, éventuellement en présence d'un agent liant d'hydracide et éventuellement d'un solvant inerte à l'égard des partenaires de la réaction.

7. Procédé de préparation d'un anilide d'acide N-azolylacétique selon la revendication 6, caractérisé par le fait qu'on effectue la réaction dans un système à deux phases, l'une des phases étant constituée d'un alcali aqueux et l'autre phase d'un solvant inerte, non miscible avec la phase aqueuse dans les conditions de réaction, et on effectue la réaction en présence d'un catalyseur de transfert de phases.

8. Un anilide d'acide N-azolylacétique selon la revendication 1, à savoir 2',6'-diméthylanilide d'acide N-(1,2,4-triazol-1-yl-méthyl)-pyrazol-1-yl-acétique ou 2',6'-diméthylanilide d'acide N-(pyrazol-1-yl-méthyl)-pyrazol-1-yl-acétique.

9. Herbicide selon la revendication 2, contenant, comme composant actif, un anilide d'acide N-azolylacétique selon la revendication 1, à savoir 2',6'-diméthylanilide d'acide N-(1,2,4-triazol-1-yl-méthyl)-pyrazol-1-yl-acétique ou 2',6'-diméthylanilide d'acide N-(pyrazol-1-yl-méthyl)-pyrazol-1-yl-acétique.

**Revendications** pour l'Etat contractant: AT

1. Herbicide contenant, comme composant actif, un anilide d'acide N-azolylacétique de formule I

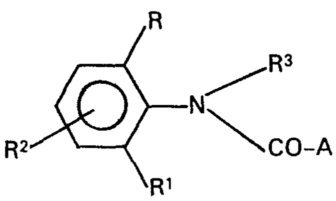

(I)

dans laquelle

R représente hydrogène ou alkyle en $C_1$ à $C_4$,

$R^1$ hydrogène, alkyle en $C_1$ à $C_4$ ou halogène,

$R^2$ hydrogène ou méthyle,

$R^3$ représente A, et

A représente un reste N-azolylméthyle qui peut être substitué dans le noyau hétérocyclique par un à trois restes du groupe méthyle, méthoxy ou chlore.

2. Herbicide contenant un support solide ou liquide et un anilide d'acide N-azolylacétique de formule I tel que défini dans la revendication 1.

3. Procédé de préparation d'un herbicide, caractérisé par le fait qu'on mélange un support solide ou liquide avec un anilide d'acide N-azolylacétique de formule I, tel que défini dans la revendication 1.

4. Procédé de lutte contre la croissance indésirable de plantes, caractérisé par le fait qu'on traite le sol ou les plantes avec un anilide d'acide N-azolylacétique de formule I, tel que défini dans la revendication 1.

5. Procédé de préparation d'un anilide d'acide N-azolylacétique de formule I, tel que défini dans la revendication 1, caractérisé par le fait qu'on fait réagir un 2-halogènacétanilide de formule II

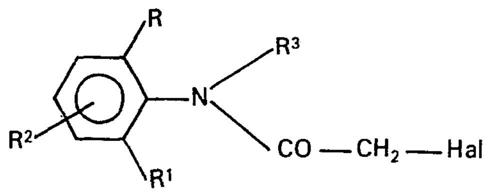

(II)

dans laquelle R, $R^1$, $R^2$ et $R^3$ ont la signification donnée à la revendication 1 et Hal représente halogène, en particulier chlore ou brome, avec un azole de formule III

M–B (III)

dans laquelle M représente hydrogène ou un métal alcalin et B un reste azole substitué par un à trois restes du groupe méthyle, méthoxy ou chlore, éventuellement en présence d'un agent-liant d'hydracide et éventuellement d'un solvant inerte à l'égard des partenaires de la réaction.

6. Procédé de préparation d'un anilide d'acide N-azolylacétique selon la revendiction 5, caractérisé par le fait qu'on effectue la réaction dans un système à deux phases, l'une des phases étant constituée d'un alcali aqueux et l'autre phase d'un solvant inerte, non miscible avec la phase aqueuse dans les conditions de réaction, et on effectue la réaction en présence d'un catalyseur de transfert de phases.

7. Herbicide selon la revendication 1, contenant, comme composant actif, un anilide d'acide N-azolylacétique, à savoir 2',6'-diméthylanilide d'acide N-(1,2,4-triazol-1-yl-méthyl)-pyrazol-1-yl-acétique ou 2',6'-diméthylanilide d'acide N-(pyrazol-1-yl-méthyl)-pyrazol-1-yl-acétique.